# EUROPEAN PATENT SPECIFICATION

(11) **EP 2 684 557 B1**
(45) Date of publication and mention of the grant of the patent: **26.04.2017**
(21) Application number: 12755612.4
(22) Date of filing: 11.01.2012
(51) Int. Cl.: A61K 8/46, A61Q 1/02

(54) **FOUNDATION COSMETIC AND METHOD FOR PRODUCING SAME**
BASISKOSMETIKUM UND HERSTELLUNGSVERFAHREN DAFÜR
PRODUIT COSMÉTIQUE DE TYPE FOND DE TEINT ET SON PROCÉDÉ DE PRODUCTION

(30) Priority: 08.03.2011 JP 2011050333
(43) Date of publication of application: 15.01.2014
(73) Proprietor: FUJIFILM Corporation, Tokyo 106-8620 (JP)
(72) Inventor: NAKAMURA, Kazuhiro, Ashigarakami-gun Kanagawa 258-8577 (JP)
(74) Representative: Höhfeld, Jochen
(86) International application number: PCT/JP2012/050381
(87) International publication number: WO 2012/120916

(56) References cited:
- JP-A- 3 139 569
- JP-A- 8 059 434
- JP-A- H1 135 840
- JP-A- 55 133 462
- JP-A- 56 108 703
- JP-A- H09 255 528
- JP-A- 2001 234 090
- JP-A- 2003 105 225
- JP-A- 2010 260 835

## Description

### Technical Field

The present invention relates to a base makeup cosmetic and a method of producing the same.

### Background Art

A base makeup cosmetic such as a foundation is required to have ability to cover up dullness of skin color (a state with lowered lightness and increased yellow chroma) which is perceived with aging caused by poor blood circulation or pigmentation, and to give a natural finished color tone or the like; and various base makeup cosmetics and methods of production thereof have been proposed.

For example, as a technique focused on realization of a natural and healthy skin color without dullness, a foundation having a depression in a spectrum in a wavelength region of from 500 to 620 nm in a reflectometric spectrum has been disclosed by Japanese Patent No. 3514915.

As a molding method for a base makeup cosmetic in a solid powder form, in addition to a dry molding method, a wet molding method is also used, and a solid powder foundation produced by a wet molding such that a high aspect ratio powder and a spherical powder are incorporated, in order to produce a good cosmetic without a crack by a wet molding, is disclosed in Japanese Patent No. 3675564.

A colorant used for a cosmetic and the like having various capabilities simultaneously has been proposed and, for example, Japanese Patent Application Laid-Open (JP-A) No. 2003-105225 discloses a technique in which an organic composite pigment, in which an organic dye stuff is fixed to an inorganic substance, is added in view of improvement in color development and suppression of staining of the skin.

### SUMMARY OF INVENTION

### Technical Problem

However, when a conventional base makeup cosmetic is applied to the skin, the color tone of the skin color may appear yellowish (hereinafter also referred to as "yellow dulling") depending on the type of light source. Namely, there has been a drawback in that a natural skin color finishing that is independent of a light source cannot be obtained.

Further, with respect to conventional base makeup cosmetics, a feel of use thereof (a feel in picking it up with a puff or the like, and a soft feel, a moist feel and smooth feel when applying it to the skin) is still unsatisfactory.

Furthermore, when a wet molding method is used for molding a base makeup cosmetic, there is a drawback that a pigment included in a cosmetic base material as a colorant dissolves into the solvent (bleeds-out) causing uneven coloring to impair the appearance of the cosmetic, or staining when applied to the skin.

The present invention has been made in view of aforementioned existing circumstances. An object of the present invention is to provide a base makeup cosmetic, which can give a natural skin color finishing independent of a light source, which is superior in a feel of use, and with which even though a wet molding method is used, uneven coloring on a cosmetic surface, or staining of the skin caused by bleed-out of a colorant component is suppressed, and to provide a method of production thereof.

### Solution to Problem

Exemplary Embodiments of the present invention are as follows:
[1] A base makeup cosmetic molded by filling a slurry into a container, the slurry including a solvent and a cosmetic base material including a red iron oxide and a red composite powder having a composite structure in which a red pigment selected from the group consisting of lithol rubine B and lithol rubine BCA is intercalated into an inorganic substance, and then removing the solvent,
   wherein the solvent is ethanol, water, or a combination thereof and wherein a reflectance spectrum of an apparent color of the base makeup cosmetic has a local minimal value in a wavelength region from 500 nm to 600 nm.
[2] The base makeup cosmetic according to [1], wherein the red composite powder is a red composite powder in which respective local maximal values of absorbance measured in a wavelength region from 400 nm to 600 nm of filtrates obtained in the following first dissolution test in water and the following second dissolution test in ethanol, using the red composite powder, are less than 0.10;
   wherein, in the first dissolution test:
   1 part by mass of the red composite powder is dispersed in 99 parts by mass of distilled water as a dispersion medium with shaking for 60 seconds, the dispersion is then stirred with a stirrer at 400 rpm and 40°C for 6 hours, and thereafter is filtrated by a cellulose acetate filter with a pore size of 0.45 µm to obtain a filtrate; and an absorbance of the obtained filtrate is measured using distilled water as a blank to determine a local maximal value of absorbance in a wavelength region from 400 nm to 600 nm; and
   wherein, in the second dissolution test:
   1 part by mass of the red composite powder is dispersed in 99 parts by mass of 99.5 v/v % absolute ethanol with shaking for 60 seconds, the dispersion is then stirred with a stirrer at 400 rpm and 40°C for 6 hours, and thereafter is filtrated by a cellulose acetate filter with a pore size of 0.45 µm to obtain a filtrate; and an absorbance of the obtained filtrate is measured using ethanol as a blank to determine a local maximal value of absorbance in a wavelength region from 400 nm to 600 nm.
[3] The base makeup cosmetic according to [1] or [2], wherein the base makeup cosmetic is a powder foundation, and a hue angle h of an apparent color of the base makeup cosmetic, measured with standard light from a D65 light source, is in a range from 50° to 80°.
[4] The base makeup cosmetic according to any one of [1] to [3], wherein the base makeup cosmetic is a pressed powder, and a hue angle h of an apparent color of the base makeup cosmetic, measured with standard light from a D65 light source, is in a range from -90° to 90°.
[5] A method of producing a base makeup cosmetic, the method including preparing a slurry by mixing a solvent and a cosmetic base material including a red iron oxide and a red composite powder having a composite structure in which a red pigment selected from the group consisting of lithol rubine B and lithol rubine BCA is intercalated into an inorganic substance, and filling the slurry into a container and then removing the solvent, wherein the solvent is ethanol, water, or a combination thereof and wherein a reflectance spectrum of an apparent color of the base makeup cosmetic has a local minimal value in a wavelength region from 500 nm to 600 nm.
[6] The method of producing a base makeup cosmetic according to [5], wherein the red composite powder is a red composite powder in which respective local maximal values of absorbance measured in a wavelength region from 400 nm to 600 nm of filtrates obtained by the following first dissolution test in water and the following second dissolution test in ethanol, using the red composite powder, are less than 0.10;
   wherein, in the first dissolution test:
   1 part by mass of the red composite powder is dispersed in 99 parts by mass of distilled water as a dispersion medium with shaking for 60 seconds, the dispersion is then stirred with a stirrer at 400 rpm and 40°C for 6 hours, and thereafter is filtrated by a cellulose acetate filter with a pore size of 0.45 µm to obtain a filtrate; and an absorbance of the obtained filtrate is measured using distilled water as a blank to determine a local maximal value of absorbance in a wavelength region from 400 nm to 600 nm; and
   wherein, in the second dissolution test:
   1 part by mass of the red composite powder is dispersed in 99 parts by mass of 99.5 v/v % absolute ethanol with shaking for 60 seconds, the dispersion is then stirred with a stirrer at 400 rpm and 40°C for 6 hours, and thereafter is filtrated by a cellulose acetate filter with a pore size of 0.45 µm to obtain a filtrate; and an absorbance of the obtained filtrate is measured using ethanol as a blank to determine a local maximal value of absorbance in a wavelength region from 400 nm to 600 nm.
[7] The method of producing a base makeup cosmetic according to [5] or [6], wherein the base makeup cosmetic is a powder foundation, and a hue angle h of an apparent color of the base makeup cosmetic, measured with standard light from a D65 light source, is in a range from 50° to 80°.
[8] The method of producing a base makeup cosmetic according to any one of [5] to [7] above, wherein the base makeup cosmetic is a pressed powder, and a hue angle h of an apparent color of the base makeup cosmetic, measured with standard light from a D65 light source, is in a range from -90° to 90°.

### Advantageous Effects of Invention

According to the present invention, a base makeup cosmetic, which can give a natural skin color finishing independent of a light source, which is superior in a feel of use, and with which, even though a wet molding method is used, uneven coloring on a cosmetic surface or staining of the skin caused by bleed-out of a colorant component is suppressed, as well as a method of production thereof, can be provided.

### BRIEF DESCRIPTION OF DRAWINGS

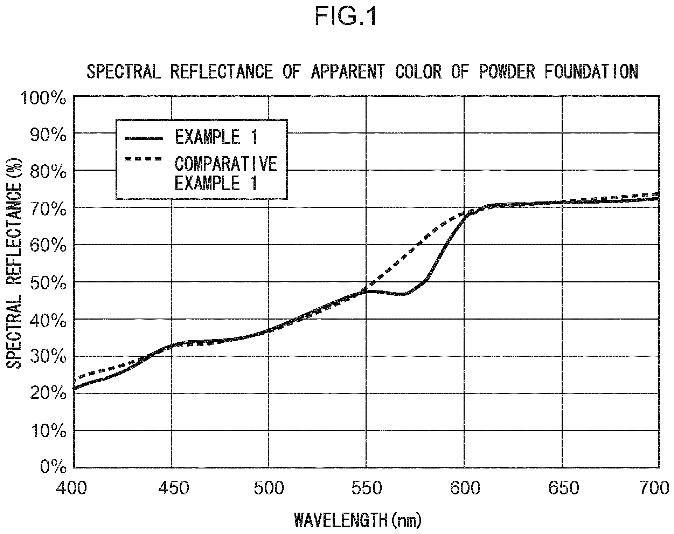

Figure 1 is a graph showing the spectral reflectance (%) of the apparent color of the powder foundation obtained in each of Example 1 and Comparative Example 1.

### DESCRIPTION OF EMBODIMENTS

The base makeup cosmetic and the method of production therefor according to the present invention are described below in details.

In the present specification, a numerical range expressed using "to" means a range including the numerical values before and after "to" as the minimum and maximum values, respectively.

In a case in which the amount of a component in the composition is indicated in the invention, when there are plural substances corresponding to the component in the composition, the indicated amount means the total amount of the plural substances present in the composition, unless specifically stated otherwise.

In the present specification, the term "process" encompasses an independent process, as well as a process that cannot be clearly distinguished from another process but yet achieves the expected effect of the process of interest.

### [Base makeup cosmetic and method of production therefor]

The base makeup cosmetic according to the present invention (hereinafter sometimes referred to as "cosmetic") is a base makeup cosmetic obtained by preparing a slurry including a solvent and a cosmetic base material including a red iron oxide and a red composite powder (hereinafter sometimes referred to as "specific red composite powder") having a composite structure in which a red pigment selected from the group consisting of lithol rubine B and lithol rubine BCA is intercalated into an inorganic substance, and molding by filling the slurry into a container, and then removing the solvent.

The scope of the meaning of the term "fill" encompasses herein not only a case in which a container is made full with a content, but also a case in which a container is loaded to a desired level.

Namely, the cosmetic according to the present invention is obtained by a method of production including preparing a slurry by mixing a solvent and a cosmetic base material including a red iron oxide and a red composite powder having a composite structure in which a red pigment selected from the group consisting of lithol rubine B and lithol rubine BCA is intercalated into an inorganic substance, and filling the slurry into a container and then removing the solvent (a method of production according to the present invention).

The cosmetic according to the present invention can yield a natural skin color finishing independent of a light source, for example, improving yellow dulling, can be superior in a feel of use, and, even though a wet molding method is applied, can efficiently suppress uneven coloring on a cosmetic surface or staining of the skin caused by dissolution of a colorant component.

Examples of an application form of the base makeup cosmetic according to the present invention include a powder foundation, and a pressed powder.

### (1) Cosmetic base material

Components to be contained in the cosmetic base material of the cosmetic according to the present invention and components which may be optionally contained in the same are described.

### <Specific red composite powder>

The cosmetic according to the present invention contains a red composite powder (specific red composite powder) having a composite structure in which a red pigment selected from the group consisting of lithol rubine B and lithol rubine BCA is intercalated into an inorganic substance by intercalation. The specific red composite powder can function as a colorant component in the cosmetic according to the present invention. The specific red composite powder is herein included in the concept of a red pigment.

Lithol rubine B and lithol rubine BCA, which are components of the specific red composite powder, are monoazo type red pigments, the former being known as Red No. 201, and the latter being known as Red No. 202. The specific composite powder in the present invention has a composite structure formed by intercalation of the red pigment. Here, intercalation or to be intercalated means a phenomenon in which a molecule, an atom or an ion is inserted between layers of a substance having a layered structure.

More specifically, the specific red composite powder according to the present invention is obtained by forming a composite of a red pigment selected from lithol rubine B or lithol rubine BCA with a planar layered inorganic powder and two or more kinds of inorganic hydroxides thereby fixing.

The specific red composite powder can be obtained by once completely dissolving a red pigment selected from the group consisting of lithol rubine B and lithol rubine BCA in water, separating two kinds of inorganic hydroxides out in the presence of two kinds of inorganic salts while adjusting the pH, adding a planar layered inorganic powder, and forming a composite of the organic dye stuff, the two kinds of inorganic hydroxides, and the planar layered inorganic powder, thereby chemically fixing. In particular, the production can be made according to the method of production described in JP-A No. 2003-105225.

The content of pure dye stuff component in a specific red composite powder is preferably 5 % by mass or more but less than 50 % by mass, and more preferably from 10 % by mass to 30 % by mass, from viewpoints of color development and suppression of a dye stuff component into a solvent.

Examples of the inorganic salt include a metallic chloride, such as magnesium chloride, and aluminum chloride. Examples of the inorganic hydroxide include magnesium hydroxide and aluminum hydroxide. Examples of the planar layered inorganic powder include montmorillonite, beidellite, hectorite, saponite, nontronite, and an expansive fluorine-bearing mica.

As for the particle diameter of the specific red composite powder, the average primary particle diameter is preferably from 1 µm to 10 µm, and more preferably from 2 µm to 8 µm.

From a viewpoint of suppression of dissolution into a solvent (bleed-out), the specific red composite powder is preferably a red composite powder in which respective local maximal values of absorbance measured in a wavelength region from 400 nm to 600 nm of filtrates obtained by the following first dissolution test specified below in water and the following second dissolution test in ethanol, using the specific red composite powder, are less than 0.10.

The local maximal values of absorbance are more preferably less than 0.01, and the lower, the more preferable.

### First dissolution test:

One part by mass of the specific red composite powder is dispersed in 99 parts by mass of distilled water as a dispersion medium with shaking for 60 seconds. The dispersion is then stirred with a stirrer at 400 rpm and 40°C for 6 hours, and thereafter is filtrated by a cellulose acetate filter with the pore size of 0.45 µm (trade name: CELLULOSE ACETATE, manufactured by ADVANTEC) to obtain a filtrate. The absorbance of the obtained filtrate is measured using distilled water as a blank to determine a local maximal value of absorbance in a wavelength region from 400 nm to 600 nm.

### Second dissolution test:

One part by mass of the red composite powder is dispersed in 99 parts by mass of 99.5 v/v % absolute ethanol with shaking for 60 seconds. The dispersion is then stirred with a stirrer at 400 rpm and 40°C for 6 hours, and thereafter is filtrated by a cellulose acetate filter with the pore size of 0.45 µm (Trade name: CELLULOSE ACETATE, manufactured by ADVANTEC) to obtain a filtrate. The absorbance of the obtained filtrate is measured using ethanol as a blank to determine a local maximal value of absorbance in a wavelength region from 400 nm to 600 nm.

For measuring the absorbance in the first dissolution test and the second dissolution test, a spectrophotometer UV-2550 (Trade name, by Shimadzu Corporation) was used.

As a specific red composite powder, a commercial product can be used and examples thereof include HNB RED7, and HNB RED6 (trade names, by Daito Kasei Kogyo Co., Ltd.

The content of the specific red composite powder in a cosmetic according to the present invention is required to be 10 % by mass or more with respect to the total mass of red pigment required to adjust the color of a cosmetic to a targeted color, and is preferably 50 % by mass or more, more preferably 75 % by mass or more, and further preferably 100 % by mass.

The content of the specific red composite powder is adjusted within the above range considering the color forming efficiency of a relevant type.

The cosmetic according to the present invention may contain a single kind of specific red composite powder, or 2 or more kinds.

### <Other components>

The cosmetic base material in the cosmetic according to the present invention may contain, according to need, in addition to the specific red composite powder, one or more additional components, to the extent that advantageous effects of the present invention are not impaired. Examples of the additional components include a pigment, such as an extender pigment, a coloring pigment, or a pearl pigment, an oil, and other additives, however the additional components are not limited thereto. The additional components preferably contained in the cosmetic according to the present invention are described below.

### <<Extender pigment>>

It is preferable that the cosmetic according to the present invention further contain an extender pigment. The extender pigment means a pigment that does not substantially contribute to adjustment of a hue.

Examples of the extender pigment include mica, synthetic phlogopite, talc, kaolin, mica, sericite, manganese carbonate, calcium carbonate, silicic anhydride, aluminum oxide, and barium sulfate.

As the extender pigment, a commercial product may also be used, and examples thereof include SERICITE FSE (by Sanshin Mining Ind. Co., Ltd.), TALK JA-46R (by Asada Milling Co., Ltd.), and a synthetic phlogopite PDM series (by Topy Industries Ltd.).

As for the particle diameter of the extender pigment, its mean primary particle size is preferably from 1 µm to 100 µm, and more preferably from 5 µm to 80 µm.

The particle size of a pigment according to the present invention can be measured by preparing a dispersion in a solvent containing the pigment which is a measurement object at a given concentration, and measuring the same by any of various commercial measurement instruments based on a principle of laser light scattering (for example, a laser diffraction scattering particle size distribution analyzer LMS-30 (trade name, manufactured by Seishin Enterprise Co., Ltd.)).

The extender pigment is preferably contained in the cosmetic preferably as a major component, and the content thereof is preferably from 50 % by mass to 90 % by mass with respect to the total mass of the cosmetic.

The cosmetic according to the present invention may contain one kind of extender pigment singly, or two or more kinds thereof.

### <<Coloring pigment>>

It is preferable that the cosmetic according to the present invention further contain a coloring pigment as a colorant component other than the specific red composite powder. The coloring pigment means a pigment that contributes to adjustment of a hue and that is not a pearl pigment.

Examples of the coloring pigment include an iron oxide, such as yellow iron oxide, red iron oxide, and black iron oxide, a white pigment, and a certified color, such as, Red No. 226, Yellow No. 4, Yellow No. 5, and Yellow No. 401.

Among the coloring pigments, from viewpoints of color tone adjustment and suppression of dissolution into a solvent (bleed-out), an iron oxide is preferable, and red iron oxide is more preferable.

When, as a white pigment, titanium oxide, zinc oxide or the like is contained, the white pigment may also have a function as a masking agent against blemishes, freckles or the like, or an ultraviolet ray protecting agent.

With respect to the shape and the particle diameter of the coloring pigments, for example, with respect to the shape and the particle diameter of a white pigment, a white pigment which is spherical and from several nm to several hundreds nm is preferably used, and with respect to the shape and the particle diameter of an iron oxide, an iron oxide which is spherical or acicular and from several nm to several hundreds nm is preferably used.

The addition amounts of the coloring pigments are adjusted appropriately so as to attain the color tone of the entire cosmetic to the target tone.

The cosmetic according to the present invention may contain one kind of coloring pigment singly, or two or more kinds thereof.

### <<Pearl pigment>>

It is preferable that the cosmetic according to the present invention further contain a pearl pigment. The pearl pigment means a pigment that contributes to adjustment of a hue and that has a pearly luster.

Examples of the pearl pigment include titanium oxide-coated mica (titanated mica), titanium oxide-coated glass flake, and titanium oxide-coated talc. A pearl pigment in which the titanium oxide coating has a multiple layer structure, or a multiple layer structure including a silicon oxide layer, may also be preferably used.

As a pearl pigment, a commercial product may be used, and examples thereof include RONAFLAIR BALANCE GOLD, TRANS PRISMA RED, TIMIRON SUPER SILK MP-1005 (All of them are trade names, manufactured by MERCK), and FLAMENCO series (trade name, manufactured by BASF).

As the pearl pigment, a gold pearl pigment and a red pearl pigment are preferable, and the pearl pigment is also preferably a mixture.

As for the particle diameter of the pearl pigment, its mean primary particle diameter is preferably from 0.5 µm to 100 µm, and more preferably from 1 µm to 80 µm.

The content of the pearl pigment with respect to the total mass of the cosmetic is preferably from 1 % by mass to 50 % by mass, more preferably from 5 % by mass to 30 % by mass, and further preferably from 7.5 % by mass to 15 % by mass.

The cosmetic according to the present invention may contain one kind of pearl pigment singly, or two or more kinds thereof, and is preferably added in such a ratio that the hue angle of the reflectance color of the entire pearl pigment contained in the cosmetic is within a range from 40° to 80°.

Examples of a preferable combination of the specific red composite powder and the above pigments to be used jointly include a combination of at least one kind of extender pigment selected from mica, sericite, and talc, at least one kind of coloring pigment selected from yellow iron oxide, red iron oxide, black iron oxide, and titanium oxide, and at least one kind of pearl pigment selected from a gold pearl pigment and a red pearl pigment.

### <<Oil>>

It is preferable that the cosmetic according to the present invention further contain an oil.

Examples of the oil include oils commonly used in a cosmetic, such as a liquid paraffin, a vaseline, a paraffin wax, squalane, a beeswax, carnauba wax, an olive oil, lanolin, a higher alcohol, a fatty acid, a higher fatty acid, an ester oil, ceresin, a microcrystalline wax, candelilla wax, a diglyceride, a triglyceride, a silicone oil, perfluoropolyether, perfluorodecalin, perfluorooctane, a jojoba oil, octyldodecyl myristate, and neopentylglycol dioctanoate.

### <<Other additives>>

A cosmetic according to the present invention may contain, according to need, a component usually added to a cosmetic, such as a surfactant, a water-soluble polymer, a powder other than the specific red composite powder and the pigments described above respectively, a humectant, an antiseptic agent, a medicinal ingredient, an ultraviolet absorber, a dye stuff, an inorganic salt or an organic salt, a fragrance, a chelating reagent, a pH adjuster, and water, to the extent advantageous effects of the present invention are not impaired.

Examples of the surfactant include surfactants commonly used in a cosmetic involving a nonionic surfactant, such as a polyoxyethylene alkyl ether, a polyoxyethylene fatty acid ester, a polyoxyethylene sorbitan fatty acid ester, a glycerol fatty acid ester, a polyglycerol fatty acid ester, a polyoxyethylene glycerol fatty acid ester, a polyoxyethylene hydrogenated castor oil, and a polyoxyethylene sorbitol fatty acid ester; an anionic surfactant as represented by a fatty acid soap, such as sodium stearate, and triethanolamine palmitate; a cationic surfactant; and an amphoteric surfactant.

Examples of the water-soluble polymer include water-soluble polymers commonly used in a cosmetic, such as carboxymethyl cellulose, methyl cellulose, hydroxymethyl cellulose, polyvinyl alcohol, polyvinylpyrrolidone, tragacanth gum, carrageenan, locust bean gum, dextrin, a dextrin fatty acid ester, a carboxyvinyl polymer, xanthan gum, gelatin, sodium alginate, gum arabic, and water-soluble collagen.

Examples of the humectant include humectants commonly used in a cosmetic, such as sorbitol, xylitol, glycerol, maltitol, propylene glycol, 1,3-butylene glycol, 1,4-butylene glycol, sodium pyrrolidone carboxylate, lactic acid, sodium lactate, and polyethylene glycol.

Examples of the antiseptic agent include antiseptic agents commonly used in a cosmetic, such as a *p*-oxybenzoic acid alkyl ester, sodium benzoate, and potassium sorbate.

Examples of the medicinal ingredient include ingredients commonly used in a cosmetic, such as vitamins, a herbal medicine, an anti-inflammatory agent, and an antimicrobial agent.

Examples of the ultraviolet absorber include ultraviolet absorbers commonly used in a cosmetic, such as a *p*-aminobenzoic acid type ultraviolet absorber, an anthranil type ultraviolet absorber, a salicylic acid type ultraviolet absorber, a cinnamic acid type ultraviolet absorber, and a benzophenone type ultraviolet absorber.

Examples of the dye stuff include a dye stuff applicable to a cosmetic such as a natural dye stuff, examples thereof including a *Haematococcus* algae dye stuff.

Examples of the inorganic salt or the organic salt include an alkali metal salt, an alkaline-earth metal salt, or an aluminum salt of an inorganic acid, such as hydrochloric acid, sulfuric acid, and nitric acid; an oxycarboxylic acid, such as citric acid, tartaric acid, lactic acid, and malic acid; a carboxylic acid, such as formic acid, acetic acid, and sorbic acid; and an aromatic carboxylic acid, such as salicylic acid, and benzoic acid.

Specific examples of a preferable inorganic salt or organic salt include potassium sulfate, sodium sulfate, magnesium sulfate, aluminum sulfate, potassium nitrate, sodium nitrate, magnesium nitrate, aluminum nitrate, calcium nitrate, potassium chloride, magnesium chloride, sodium chloride, calcium chloride, aluminum chloride, potassium carbonate, sodium carbonate, aluminum carbonate, potassium acetate, sodium acetate, calcium acetate, magnesium acetate, sodium formate, potassium formate, magnesium formate, sodium citrate, sodium tartrate, potassium sorbate, sodium sorbate, sodium salicylate, potassium benzoate, and sodium benzoate; and especially potassium sulfate, magnesium sulfate, potassium chloride, magnesium chloride, aluminum chloride, sodium citrate, sodium tartrate, potassium sorbate, sodium salicylate and sodium benzoate are preferable.

The inorganic salt or the organic salt may be added to the cosmetic according to the present invention in a form of a salt; or may be produced in producing the cosmetic according to the present invention by adding a corresponding acid substance and a base substance in a stoichiometric amount required for forming a salt. Water may be added in an arbitrary amount.

### (2) Production of cosmetic

The cosmetic according to the present invention is produced by a method of production according to the present invention, which includes preparing a slurry by mixing a solvent and a cosmetic base material including a red composite powder (specific red composite powder) having a composite structure in which a red pigment selected from the group consisting of lithol rubine B and lithol rubine BCA is intercalated into an inorganic substance (hereinafter sometimes referred to as "slurry preparation process"), and filling the slurry into a container and then removing the solvent (hereinafter sometimes referred to as "molding process").

The method of production applied to the cosmetic according to the present invention is a wet molding method, by which a superior feel of use can be achieved.

### (Slurry preparation process)

In this process, the cosmetic base material containing a specific red composite powder and other components which may be optionally used, is mixed with a solvent to obtain a slurry. Details of the specific red composite powder and other components contained in a cosmetic base material are as described above.

The slurry is preferably prepared by mixing and pulverizing respective components of the cosmetic base material to yield a mixture, and mixing the mixture in a solvent with stirring to a homogeneous state.

The components of the cosmetic base material may be mixed by, for example, a HENSCHEL mixer, until the hue of the mixture becomes homogeneous.

### <<Solvent>>

Examples of the solvent to be used in this process include water, ethanol, hexane, light liquid isoparaffin, isododecane, and a mixture thereof.

Among the above solvents, from a viewpoint of improvement of a feel of use, ethanol, water, and a mixture thereof are preferable.

The content of the solvent in a slurry is preferably from 5 % by mass to 50 % by mass, and more preferably from 10 % by mass to 30 % by mass with respect to the total mass of the cosmetic base material.

### (Molding process)

In this process, the slurry obtained in the slurry preparation process is filled in a container, and then the solvent is removed.

Examples of the container to fill the slurry include various dish-shaped containers commonly used for a base makeup cosmetic.

Examples of a method for removing the solvent include a method in which after filling the slurry in the container, compression molding is carried out while suction is carried out through a porous suction head, and then drying is carried out.

Although the drying temperature varies depending on the volatility of, for example the oil added in the cosmetic, it is preferably from 20°C to 80°C, and more preferably from 30°C to 70°C. The drying time is preferably from 5 min to 24 hours, and more preferably from 1 hour to 6 hours.

A cosmetic according to the present invention obtained as above preferably exhibits a reflectance spectrum of the apparent color thereof having a local minimal value in a wavelength region from 500 nm to 600 nm from a viewpoint of suppression of yellow dulling.

The reflectance spectrum herein is a value obtained by measurement by SPECTROLINO (trade name) (manufactured by Gretag Machbeth).

In a case where the cosmetic according to the present invention is a powder foundation, which is a preferable embodiment, the hue angle h of the apparent color of the cosmetic, measured with standard light from a D65 light source, is preferably in a range from 50° to 80°, and more preferably in a range from 55 to 75.

In a case where the cosmetic according to the present invention is a pressed powder, the hue angle h of the apparent color of the cosmetic, measured with standard light from a D65 light source, is preferably in a range from -90° to 90°, and more preferably in a range from -60° to 60°.

The hue angle h of the apparent color in the present invention is a value determined by measurements of spectral reflectances over wavelengths from 380 nm to 730 nm using a spectrophotometer SPECTROLINO (Trade name, by Gretag Machbeth), and calculation from a* value and b* value of reflected light assuming a D65 light source in accordance with the definition of the L*a*b* color space (CIE 1976).

### EXAMPLES

The present invention is described below by way of Examples in details, provided that the present invention be not limited thereto by any means. The "part" herein is by mass unless otherwise specified.

### [Examples 1 to 4, and Comparative Examples 1, 2 and 4]

### <Preparation of powder foundation>

The respective components described in A Phase and B Phase in Table 1 (numerical values are expressed in part by mass) were weighed out individually, mixed and pulverized in a HENSCHEL mixer to yield a mixture. Then, the mixture was added into a 60 parts by mass of a water/ethanol mixed solvent (mixing ratio by mass was water : ethanol = 25 : 75) with stirring, and mixed until uniform, whereby a slurry was obtained.

Then the yielded slurry was filled in a gold dish, and compression molding was carried out with suction through a porous suction head. Thereafter, the product was dried at a temperature of 37°C for 24 hours to prepare each of powder foundations of Examples 1 to 4, and Comparative Examples 1, 2 and 4.

### [Example 5]

The respective components described in A Phase and B Phase in Table 1 (numerical values are expressed in part by mass) were weighed out individually, and mixed in a HENSCHEL mixer. Thereafter, 0.01 part by mass of water-soluble collagen (trade name: COLLAGEN P, manufactured by Nitta Gelatin Inc., concentration of 1 % by mass) and 0.01 part by mass of a *Haematococcus* algae dye stuff (trade name: ASTOTS 50, manufactured by Takeda Shiki Colk Ltd., Astaxanthin concentration: 5 % by mass) were further mixed thereto in the HENSCHEL mixer until the resulting mixture becomes uniform, whereby a slurry was obtained.

Then, in the same manner as Examples 1 to 4, the obtained slurry was filled in a gold dish, compression molding with suction was carried out, and drying was carried out, whereby a foundation of Examples 5 was prepared.

### [Comparative Examples 3 and 5]

Powder foundations of Comparative Examples 3 and 5 were prepared in the same manner as in Example 1 except that the slurry preparation and the filling of the slurry in a gold dish in the preparation of a powder foundation in Example 1 were not carried out, but by a dry pressing process, a mixture was filled into a gold dish and compression molding was carried out.

**[Table 1]**

| (part by mass) | | | | | | | | | | | |
|---|---|---|---|---|---|---|---|---|---|---|---|
| | Material identity | Example 1 | Example 2 | Example 3 | Example 4 | Example 5 | Comparative Example 1 | Comparative Example 2 | Comparative Example 3 | Comparative Example 4 | Comparative Example 5 |
| A Phase | Extender pigment: Sericite ^{*1} | 39.8 | 39.9 | 40.0 | 53.3 | 39.9 | 54.0 | 54.1 | 54.1 | 53.8 | 39.8 |
| | Extender pigment: Talc^{*2} | 18.0 | 18.0 | 18.0 | 24.0 | 18.0 | 24.3 | 24.4 | 24.4 | 24.3 | 18.0 |
| | Coloring pigment: Titanium ^{*3} oxide | 9.0 | 9.0 | 9.0 | 9.0 | 9.0 | 9.0 | 9.0 | 9.0 | 9.0 | 9.0 |
| | Coloring pigment: Iron oxide (yellow) ^{*4} | 2.255 | 2.255 | 2.255 | 2.255 | 2.255 | 1.804 | 2.255 | 2.255 | 1.804 | 2.255 |
| | Coloring pigment: Iron oxide ^{*5} (red) | 0.000 | 0.135 | 0.271 | 0.000 | 0.135 | 0.541 | 0.000 | 0.000 | 0.000 | 0.000 |
| | Coloring pigment: Iron oxide (black)^{*6} | 0.271 | 0.271 | 0.271 | 0.271 | 0.271 | 0.271 | 0.135 | 0.135 | 0.271 | 0.271 |
| | Pearl pigment (gold)^{*7} | 13.1 | 13.1 | 13.1 | 0.0 | 13.1 | 0.0 | 0.0 | 0.0 | 0.0 | 13.1 |
| | Pearl pigment (red)^{*8} | 6.5 | 6.5 | 6.5 | 0.0 | 6.5 | 0.0 | 0.0 | 0.0 | 0.0 | 6.5 |
| | Specific red composite powder^{*9} | 1.082 | 0.812 | 0.541 | 1.082 | 0.812 | 0.000 | 0.000 | 0.000 | 0.000 | 1.082 |
| | Red pigment-PMMA composite powder^{*10} | 0.000 | 0.000 | 0.000 | 0.000 | 0.000 | 0.000 | 0.000 | 0.000 | 0.812 | 0.000 |
| | Red No. 202^{*11} | 0.000 | 0.000 | 0.000 | 0.000 | 0.000 | 0.000 | 0.108 | 0.108 | 0.000 | 0.000 |
| B Phase | Dimethicone and trimethylsiloxysilicate^{*12} | 3.0 | 3.0 | 3.0 | 3.0 | 3.0 | 3.0 | 3.0 | 3.0 | 3.0 | 3.0 |
| | Dimethicone (20)^{*13} | 7.0 | 7.0 | 7.0 | 7.0 | 7.0 | 7.0 | 7.0 | 7.0 | 7.0 | 7.0 |

| | | | | | | | | | | | |
|---|---|---|---|---|---|---|---|---|---|---|---|
| *1 OTS-2 SERICITE FSE (by Daito Kasei Kogyo Co., Ltd.) *2 OTS-2 TALK JA-46R (by Daito Kasei Kogyo Co., Ltd.) *3 OTS-2 TiO₂ CR-50 (by Daito Kasei Kogyo Co., Ltd.) *4 OTS-2 YELLOW LLXLO (by Daito Kasei Kogyo Co., Ltd.) *5 OTS-2 RED R-516L (by Daito Kasei Kogyo Co., Ltd.) *6 OTS-2 BLACK BL-100 (by Daito Kasei Kogyo Co., Ltd.) *7 RONAFLAIR BALANCE Gold (by Merck & Co., Inc.) *8 TRANS PRISMA RED (by Merck & Co., Inc.) *9 HNB RED7 (by Daito Kasei Kogyo Co., Ltd.) *10 3D-TECH PW (OTS LC3079-10)XP (by Daito Kasei Kogyo Co., Ltd.) * 11 Red No. 202 (Kishi Kasei Co., Ltd.) *12 DC593 (by Dow Coming Toray Co., Ltd.) * 13 SH200C-20cs (by Dow Coming Toray Co., Ltd.) | | | | | | | | | | | |

### <Dissolution test on red colorant component>

Dissolution tests in water and dissolution tests in ethanol on the following red colorant components used in Examples 1 to 5, and Comparative Examples 1 to 5 were conducted as follows.

### 1. Dissolution test in water

One part by mass of a red colorant component is dispersed in 99 parts by mass of distilled water (a dispersion medium) with shaking for 60 seconds. The dispersion is then stirred with a stirrer at 400 rpm and 40°C for 6 hours, and thereafter is filtrated by a cellulose acetate filter with the pore size of 0.45 µm (trade name: CELLULOSE ACETATE, manufactured by ADVANTEC GROUP) to obtain a filtrate. The absorbance of the obtained filtrate is measured using distilled water as a blank to determine a local maximal value of absorbance in a wavelength region from 400 nm to 600 nm.

### 2. Dissolution test in ethanol

One part by mass of a red colorant component is dispersed in 99 parts by mass of absolute ethanol (99.5 v/v%) with shaking for 60 seconds. The dispersion is then stirred at 400 rpm and 40°C for 6 hours, and thereafter is filtrated by a cellulose acetate filter with the pore size of 0.45 µm (trade name: CELLULOSE ACETATE, manufactured by ADVANTEC GROUP) to obtain a filtrate. The absorbance of the obtained filtrate is measured using ethanol as a blank to determine a local maximal value of absorbance in a wavelength region from 400 nm to 600 nm.

For measurement of absorbance in the above dissolution tests, a spectrophotometer (trade name: UV-2550, manufactured by Shimadzu Corporation) was used.

Red colorant components of Examples 1 to 5, and Comparative Examples 1 to 5 used as objects of the dissolution tests were summarized below. In this regard, details of the respective red colorant components are described as remarks outside the box of Table 1.
Example 1: red composite powder 1
Example 2: red composite powder 1 / iron oxide (red)
Example 3: red composite powder 1 / iron oxide (red)
Example 4: red composite powder 1
Example 5: red composite powder 1 / iron oxide (red)
Comparative Example 1: iron oxide (red)
Comparative Example 2: Red No. 202
Comparative Example 3: Red No. 202
Comparative Example 4: composite powder of red pigment and polymethyl methacrylate (PMMA)
Comparative Example 5: red composite powder 1

The results of the respective dissolution tests are shown in the following Table 2. The results are also shown in Table 3.

**[Table 2]**

| | Dissolution test | |
|---|---|---|
| | Water | Ethanol |
| Red composite powder 1 | 0.007 | 0.082 |
| Coloring pigment: iron oxide (red) | 0.001 | 0.001 |
| Red pigment-PMMA composite powder | 0.380 | 0.710 |
| Red No. 202 | 2.220 | 1.225 |

### <Evaluation of powder foundation>

### (1) Appearance

With respect to each apparent color of the obtained powder foundations of Examples 1 to 5, and Comparative Examples 1 to 5, the hue angle h was determined by measurements of spectral reflectances over wavelengths from 380 nm to 730 nm using a spectrophotometer SPECTROLINO (trade name, manufactured by Gretag Machbeth), and calculation from a* value and b*value of reflected light assuming a D65 light source in accordance with the definition of the L*a*b* color space (CIE 1976). The results are shown in Table 3.

### (2) Improvement of yellow dulling

Each of the powder foundations obtained in Example 1 to 5 or Comparative Example 1 to 5 was applied by a puff to the face of the same female test subject, and the color tone of the skin color after the application was visually observed under a three wavelength fluorescent light with the color temperature of 5000K. Then the female test subject was moved to a place in direct sunlight (midday sunlight under a clear sky, 5000K), and the relevant color tone change of the skin color was visually observed and evaluation was performed according to the following evaluation criteria. Good rating in this evaluation means that natural skin color results can be obtained independent of a light source. The results are shown in Table 3.

### -Evaluation criteria-

S: Changed clearly to red tone (no yellow dulling)
A: Changed slightly to red tone (suppressed yellow dulling)
B: No change or slight change to yellow tone
C: Changed to yellowish tone

### (3) Feel of use

With respect to each of the obtained powder foundations of Examples 1 to 5, and Comparative Examples 1 to 5, overall evaluation of a feeling in picking it up with a puff, and a soft feel, a moist feel, and a slick feel in applying it to the skin was conducted as per the following in 3 grades. The results are shown in Table 3.

### -Evaluation criteria-

A: Good in all the feelings
B: Overall, ordinary level in feeling
C: Poor in any of feelings

### (4) Bleed-out property

With respect to each of the obtained powder foundations after molding of Examples 1 to 5, and Comparative Examples 1 to 5, existence of unevenness of red tone on the cake surface or in the rim of a gold dish was examined visually and rated according to the following evaluation criteria. The results are shown in Table 3.

### -Evaluation criteria-

A: Absolutely no unevenness of red tone
B: Only slight unevenness of red tone recognized
C: Remarkable unevenness of red tone recognized

### (5) Staining on skin

After 6 hours from putting each of the obtained powder foundations of Examples 1 to 5, and Comparative Examples 1 to 5 on the skin, the foundation was rinsed off with water without using a facial cleanser. Thereafter the degree of staining of a red pigment on the skin was visually observed and evaluated according to the following evaluation criteria. The results are shown in Table 3.

### -Evaluation criteria-

A: Absolutely no red staining recognized
B: Slight red staining recognized
C: Red staining was recognized easily visually

### (6) Sweat bleeding

Each of the obtained powder foundations of Examples 1 to 5, and Comparative Examples 1 to 5 was put on the face and a synthetic sweat was sprayed on the surface, and the bleeding of the red color was visually observed and rated according to the following evaluation criteria. The results are shown in Table 3.

### -Evaluation criteria-

A: Absolutely no red bleeding recognized
B: Slight red bleeding recognized
C: Red bleeding was recognized easily visually

| [Table 3] | Red colorant component | | | Molding method | Evaluation results | | | | | |
|---|---|---|---|---|---|---|---|---|---|---|
| | Type | Dissolution test results | | | Apparent color angle h(°) | Yellow dullness | Feel of use | Bleed-out property | Staining | Sweat bleeding |
| | | Water | Ethanol | | | | | | | |
| Example 1 | Red composite powder 1 | 0.007 | 0.082 | Wet molding method | 68 | S | A | A | A | A |
| Example 2 | Red composite powder 1/Iron oxide (red) | 0.007/0.001 | 0.082/0.001 | Wet molding method | 68 | A | A | A | A | A |
| Example 3 | Red composite powder 1/Iron oxide (red) | 0.007/0.001 | 0.082/0.001 | Wet molding method | 68 | A | A | A | A | A |
| Example 4 | Red composite powder 1 | 0.007 | 0.082 | Wet molding method | 68 | S | A | A | A | A |
| Example 5 | Red composite powder 1/Iron oxide (red) | 0.007/0.001 | 0.082/0.001 | Wet molding method | 68 | A | A | A | A | A |
| Comparative Example 1 | Iron oxide (red) | 0.001 | 0.001 | Wet molding method | 68 | C | A | A | A | A |
| Comparative Example 2 | Red No. 202 | 2.220 | 1.225 | Wet molding method | 68 | S | A | C | C | C |
| Comparative Example 3 | Red No. 202 | 2.220 | 1.225 | Dry molding method | 68 | S | C | A | C | C |
| Comparative Example 4 | Composite powder of red pigment and PMMA | 0.380 | 0.710 | Wet molding method | 68 | A | A | C | B | B |
| Comparative Example 5 | Red composite powder1 | 0.007 | 0.082 | Dry molding method | 68 | S | C | A | A | A |

As shown in Table 3, it is shown that the powder foundations of Examples 1 to 5 are superior in a suppression effect of yellow dulling when the light source is changed form a fluorescent light to the sunlight, superior in a feel of use, able to suppress bleed-out of a red colorant component to the cake surface and the rim of a gold dish, and cause no recognizable skin staining or sweat bleeding, and thus exhibit overall superior properties.

On the other hand, the powder foundation of Comparative Example 1 containing iron oxide (red) but not a specific red composite powder, and obtained according to a wet molding method was inferior in a yellow dulling improving effect in changing a light source.

With respect to the powder foundation of Comparative Example 2 containing Red No. 202 but not a specific red composite powder, and obtained according to a wet molding method, red unevenness occurred on the cake surface and in the rim of a gold dish, and skin staining and sweat bleeding also occurred.

With respect to the powder foundation of Comparative Example 3 containing Red No. 202 but not a specific red composite powder, and obtained according to a dry molding method, a feel of use was poor, and skin staining and sweat bleeding occurred.

With respect to the powder foundation of Comparative Example 4 containing a composite powder different from a specific red composite powder, red unevenness occurred on the cake surface and in the rim of a gold dish, and skin staining and sweat bleeding occurred slightly.

With respect to the powder foundation of Comparative Example 5 containing a red composite powder 1 but obtained according to a dry molding method, a feel of use was poor.

Further, the spectral reflectance of the apparent color with respect to each of the powder foundations obtained in Example 1 and Comparative Example 1 was measured by a spectrophotometer SPECTROLINO (trade name, manufactured by Gretag Machbeth). The results are shown in Figure 1.

As shown in Figure 1, it is shown that with respect to the powder foundation of Example 1, the reflectance spectrum of the apparent color has a local minimal value in a wavelength region near 570 nm. On the other hand, it is shown that with respect to the powder foundation of Comparative Example 1, the reflectance spectrum of the apparent color does not have a minimal value in a wavelength region between 500 nm and 600 nm.

### [Example 6]

### <Preparation of pressed powder>

A pressed powder with the cake apparent color of L*=90, a* value=10, b* value=5, and hue angle h=27° determined by assuming a D65 light source in accordance with the definition of the L*a*b* color space (CIE 1976), was prepared in the same manner as Example 1 except that compared to Example 1 the content of the oil added in the B phase was decrease to 1/3, the contents of the iron oxide (yellow) and the red composite powder 1 were adjusted appropriately, the iron oxide (black) was not used, and lapis lazuli was added in an appropriate amount.

### <Evaluation>

When the obtained pressed powder was used on the skin having been applied with a commercial liquid foundation, it was confirmed that the powder was superior in a feel of use, and suppression of yellow dulling in changing a light source.

## Claims

1. A base makeup cosmetic molded by filling a slurry into a container, the slurry comprising a solvent and a cosmetic base material comprising a red iron oxide and a red composite powder having a composite structure in which a red pigment selected from the group consisting of lithol rubine B and lithol rubine BCA is intercalated into an inorganic substance, and then removing the solvent, wherein the solvent is ethanol, water, or a combination thereof and wherein a reflectance spectrum of an apparent color of the base makeup cosmetic has a local minimal value in a wavelength region from 500 nm to 600 nm.

2. The base makeup cosmetic according to claim 1, wherein the red composite powder is a red composite powder in which respective local maximal values of absorbance measured in a wavelength region from 400 nm to 600 nm of filtrates obtained in the following first dissolution test in water and the following second dissolution test in ethanol, using the red composite powder, are less than 0.10;
wherein, in the first dissolution test:
1 part by mass of the red composite powder is dispersed in 99 parts by mass of distilled water as a dispersion medium with shaking for 60 seconds, the dispersion is then stirred with a stirrer at 400 rpm and 40°C for 6 hours, and thereafter is filtrated by a cellulose acetate filter with a pore size of 0.45 µm to obtain a filtrate; and an absorbance of the obtained filtrate is measured using distilled water as a blank to determine a local maximal value of absorbance in a wavelength region from 400 nm to 600 nm; and
wherein, in the second dissolution test:
1 part by mass of the red composite powder is dispersed in 99 parts by mass of 99.5 v/v % absolute ethanol with shaking for 60 seconds, the dispersion is then stirred with a stirrer at 400 rpm and 40°C for 6 hours, and thereafter is filtrated by a cellulose acetate filter with a pore size of 0.45 µm to obtain a filtrate; and an absorbance of the obtained filtrate is measured using ethanol as a blank to determine a local maximal value of absorbance in a wavelength region from 400 nm to 600 nm.

3. The base makeup cosmetic according to either claim 1 or claim 2, wherein the base makeup cosmetic is a powder foundation, and a hue angle h of an apparent color of the base makeup cosmetic, measured with standard light from a D65 light source, is in a range from 50° to 80°.

4. The base makeup cosmetic according to any one of claims 1 to 3, wherein the base makeup cosmetic is a pressed powder, and a hue angle h of an apparent color of the base makeup cosmetic, measured with standard light from a D65 light source, is in a range from -90° to 90°.

5. A method of producing a base makeup cosmetic, the method comprising:
preparing a slurry by mixing a solvent and a cosmetic base material comprising a red iron oxide and a red composite powder having a composite structure in which a red pigment selected from the group consisting of lithol rubine B and lithol rubine BCA is intercalated into an inorganic substance, and
filling the slurry into a container and then removing the solvent, wherein the solvent is ethanol, water, or a combination thereof and wherein a reflectance spectrum of an apparent color of the base makeup cosmetic has a local minimal value in a wavelength region from 500 nm to 600 nm.

6. The method of producing a base makeup cosmetic according to claim 5, wherein the red composite powder is a red composite powder in which respective local maximal values of absorbance measured in a wavelength region from 400 nm to 600 nm of filtrates obtained by the following first dissolution test in water and the following second dissolution test in ethanol, using the red composite powder, are less than 0.10;
wherein, in the first dissolution test:
1 part by mass of the red composite powder is dispersed in 99 parts by mass of distilled water as a dispersion medium with shaking for 60 seconds, the dispersion is then stirred with a stirrer at 400 rpm and 40°C for 6 hours, and thereafter is filtrated by a cellulose acetate filter with a pore size of 0.45 µm to obtain a filtrate; and an absorbance of the obtained filtrate is measured using distilled water as a blank to determine a local maximal value of absorbance in a wavelength region from 400 nm to 600 nm; and
wherein, in the second dissolution test:
1 part by mass of the red composite powder is dispersed in 99 parts by mass of 99.5 v/v % absolute ethanol with shaking for 60 seconds, the dispersion is then stirred with a stirrer at 400 rpm and 40°C for 6 hours, and thereafter is filtrated by a cellulose acetate filter with a pore size of 0.45 µm to obtain a filtrate; and an absorbance of the obtained filtrate is measured using ethanol as a blank to determine a local maximal value of absorbance in a wavelength region from 400 nm to 600 nm.

7. The method of producing a base makeup cosmetic according to either claim 5 or claim 6, wherein the base makeup cosmetic is a powder foundation, and a hue angle h of an apparent color of the base makeup cosmetic, measured with standard light from a D65 light source, is in a range from 50° to 80°.

8. The method of producing a base makeup cosmetic according to any one of claims 5 to 7, wherein the base makeup cosmetic is a pressed powder, and a hue angle h of an apparent color of the base makeup cosmetic, measured with standard light from a D65 light source, is in a range from -90° to 90°.

## Patentansprüche

1. Basis-Makeup-Kosmetik, geformt durch Füllen einer breiigen Masse in einen Behälter, wobei die breiige Masse ein Lösungsmittel und ein kosmetisches Basismaterial enthält, das ein rotes Eisenoxid und ein rotes Verbundpulver mit einer Verbundstruktur, in der ein rotes Pigment, das aus der Gruppe bestehend aus Litholrubin B und Litholrubin BCA ausgewählt ist, in eine anorganische Substanz interkaliert ist, enthält, und danach Entfernen des Lösungsmittels, wobei das Lösungsmittel Ethanol, Wasser oder eine Kombination davon ist, und wobei ein Reflexionsspektrum einer sichtbaren Farbe der Basis-Makeup-Kosmetik in einem Wellenlängenbereich von 500 nm bis 600 nm einen lokalen Minimalwert hat.

2. Basis-Makeup-Kosmetik nach Anspruch 1, wobei das rote Verbundpulver ein rotes Verbundpulver ist, in dem entsprechende lokale Maximalwerte einer Absorption, die in einem Wellenlängenbereich von 400 nm bis 600 nm von Filtraten, die durch den folgenden ersten Löslichkeitstest in Wasser und den folgenden zweiten Löslichkeitstest in Ethanol unter Verwendung des roten Verbundpulvers erhalten werden, gemessen wird, weniger als 0,10 betragen;
wobei im ersten Löslichkeitstest:
1 Masseanteil an rotem Verbundpulver in 99 Masseanteilen destilliertem Wasser als Dispersionsmedium durch Schütteln für 60 Sekunden dispergiert wird, wobei die Dispersion danach mit einem Rührer bei 400 Umdrehungen pro Minute und 40°C für 6 Stunden gerührt wird, und danach durch einen Celluloseacetatfilter mit einer Porengröße von 0,45 µm filtriert wird, um ein Filtrat zu erhalten; und eine Absorption des erhaltenen Filtrats unter Verwendung von destilliertem Wasser als Blindwert gemessen wird, um einen lokalen Maximalwert der Absorption in einem Wellenlängenbereich von 400 nm bis 600 nm zu bestimmen; und
wobei im zweiten Löslichkeitstest:
1 Masseanteil an rotem Verbundpulver in 99 Masseanteilen eines 99,5 v/v %igen absoluten Ethanols durch Schütteln für 60 Sekunden dispergiert wird, wobei die Dispersion danach mit einem Rührer bei 400 Umdrehungen pro Minute und 40°C für 6 Stunden gerührt wird, und danach durch einen Celluloseacetatfilter mit einer Porengröße von 0,45 µm filtriert wird, um ein Filtrat zu erhalten; und eine Absorption des erhaltenen Filtrats unter Verwendung von Ethanol als Blindwert gemessen wird, um einen lokalen Maximalwert der Absorption in einem Wellenlängenbereich von 400 nm bis 600 nm zu bestimmen.

3. Basis-Makeup-Kosmetik nach entweder Anspruch 1 oder 2, wobei die Basis-Makeup-Kosmetik eine Pulver-Grundierung ist und ein Farbtonwinkel h einer sichtbaren Farbe der Basis-Makeup-Kosmetik, die mit Standardlicht einer D65 Lichtquelle gemessen wird, in einem Bereich von 50° bis 80° liegt.

4. Basis-Makeup-Kosmetik nach einem der Ansprüche 1 bis 3, wobei die Basis-Makeup-Kosmetik ein gepresstes Pulver ist, und ein Farbtonwinkel h einer sichtbaren Farbe der Basis-Makeup-Kosmetik, die mit Standardlicht einer D65 Lichtquelle gemessen wird, in einem Bereich von -90° bis 90° liegt.

5. Verfahren zur Herstellung einer Basis-Makeup-Kosmetik, wobei das Verfahren:
ein Herstellen einer breiigen Masse durch Mischen eines Lösungsmittels und eines kosmetischen Basismaterials, das ein rotes Eisenoxid und ein rotes Verbundpulver, das eine Verbundstruktur hat, in der ein rotes Pigment, das aus der Gruppe bestehend aus Litholrubin B und Litholrubin BCA ausgewählt ist, in eine anorganische Substanz interkaliert ist, enthält, und
ein Füllen der breiigen Masse in einen Behälter, und danach ein Entfernen des Lösungsmittels umfasst, wobei das Lösungsmittel Ethanol, Wasser oder eine Kombination davon ist, und wobei ein Reflexionsspektrum einer sichtbaren Farbe der Basis-Makeup-Kosmetik in einem Wellenlängenbereich von 500 nm bis 600 nm einen lokalen Minimalwert hat.

6. Verfahren zur Herstellung einer Basis-Makeup-Kosmetik nach Anspruch 5, wobei das rote Verbundpulver ein rotes Verbundpulver ist, in dem entsprechende lokale Maximalwerte einer Absorption, die in einem Wellenlängenbereich von 400 nm bis 600 nm von Filtraten, die durch den folgenden ersten Löslichkeitstest in Wasser und den folgenden zweiten Löslichkeitstest in Ethanol unter Verwendung des roten Verbundpulvers erhalten werden, gemessen wird, weniger als 0,10 betragen;
wobei im ersten Löslichkeitstest:
1 Masseanteil an rotem Verbundpulver in 99 Masseanteilen destilliertem Wasser als Dispersionsmedium durch Schütteln für 60 Sekunden dispergiert wird, wobei die Dispersion danach mit einem Rührer bei 400 Umdrehungen pro Minute und 40°C für 6 Stunden gerührt wird, und danach durch einen Celluloseacetatfilter mit einer Porengröße von 0,45 µm filtriert wird, um ein Filtrat zu erhalten; und eine Absorption des erhaltenen Filtrats unter Verwendung von destilliertem Wasser als Blindwert gemessen wird, um einen lokalen Maximalwert der Absorption in einem Wellenlängenbereich von 400 nm bis 600 nm zu bestimmen; und
wobei im zweiten Löslichkeitstest:
1 Masseanteil an rotem Verbundpulver in 99 Masseanteilen eines 99,5 v/v %igen absoluten Ethanols durch Schütteln für 60 Sekunden dispergiert wird, wobei die Dispersion danach mit einem Rührer bei 400 Umdrehungen pro Minute und 40°C für 6 Stunden gerührt wird, und danach durch einen Celluloseacetatfilter mit einer Porengröße von 0,45 µm filtriert wird, um ein Filtrat zu erhalten; und eine Absorption des erhaltenen Filtrats unter Verwendung von Ethanol als Blindwert gemessen wird, um einen lokalen Maximalwert der Absorption in einem Wellenlängenbereich von 400 nm bis 600 nm zu bestimmen.

7. Verfahren zur Herstellung einer Basis-Makeup-Kosmetik nach entweder Anspruch 5 oder 6, wobei die Basis-Makeup-Kosmetik eine Pulver-Grundierung ist und ein Farbtonwinkel h einer sichtbaren Farbe der Basis-Makeup-Kosmetik, die mit Standardlicht einer D65 Lichtquelle gemessen wird, in einem Bereich von 50° bis 80° liegt.

8. Verfahren zur Herstellung einer Basis-Makeup-Kosmetik nach einem der Ansprüche 5 bis 7, wobei die Basis-Makeup-Kosmetik ein gepresstes Pulver ist, und ein Farbtonwinkel h einer sichtbaren Farbe der Basis-Makeup-Kosmetik, die mit Standardlicht einer D65 Lichtquelle gemessen wird, in einem Bereich von -90° bis 90° liegt.

## Revendications

1. Produit cosmétique pour maquillage de base moulé en remplissant une bouillie dans un récipient, la bouillie comprenant un solvant et une matière de base cosmétique comprenant un oxyde rouge de fer et une poudre composite rouge présentant une structure composite, où un pigment rouge sélectionné parmi le groupe consistant en du rubis lithol B et du rubis lithol BCA est intercalé dans une substance inorganique, puis en éliminant le solvant, dans lequel le solvant est de l'éthanol, de l'eau ou une combinaison de ceux-ci, et dans lequel le spectre de réflexion d'une couleur apparente du produit cosmétique pour maquillage de base présente une valeur minimale locale dans une région de longueurs d'onde allant de 500 à 600 nm.

2. Produit cosmétique pour maquillage de base selon la revendication 1, dans lequel la poudre composite rouge est une poudre composite rouge, où les valeurs maximales locales respectives d'absorbance mesurées dans une région de longueurs d'onde allant de 400 nm à 600 nm de filtrats obtenus dans le premier essai de dissolution suivant dans l'eau et le second essai de dissolution suivant dans l'éthanol, à l'aide de la poudre composite rouge, sont inférieures à 0,10,
dans lequel, dans le premier essai de dissolution :
1 partie en masse de la poudre composite rouge est dispersée dans 99 parties en masse d'eau distillée sous la forme d'un milieu de dispersion en secouant pendant 60 secondes, la dispersion est ensuite agitée avec un agitateur à 400 tours/minute et 40 °C pendant 6 heures, puis est filtrée par un filtre d'acétate de cellulose présentant un diamètre de pore de 0,45 µm afin d'obtenir un filtrat, et une absorbance du filtrat obtenu est mesurée à l'aide de l'eau distillée comme blanc afin de déterminer une valeur maximale locale d'absorbance dans une région de longueurs d'onde allant de 400 nm à 600 nm, et
dans lequel, dans le second essai de dissolution :
1 partie en masse de la poudre composite rouge est dispersée dans 99 parties en masse de 99,5 v/v % d'éthanol absolu en secouant pendant 60 secondes, la dispersion est ensuite agitée avec un agitateur à 400 tours/minute et 40 °C pendant 6 heures, puis est filtrée par un filtre d'acétate de cellulose présentant un diamètre de pore de 0,45 µm afin d'obtenir un filtrat, et une absorbance du filtrat obtenu est mesurée à l'aide de l'éthanol comme blanc afin de déterminer une valeur maximale locale d'absorbance dans une région de longueurs d'onde allant de 400 nm à 600 nm.

3. Produit cosmétique pour maquillage de base selon la revendication 1 ou 2, dans lequel le produit cosmétique pour maquillage de base est un fond de teint pulvérulent, et un angle de teinte h d'une couleur apparente du produit cosmétique pour maquillage de base, mesuré avec une lumière standard provenant d'une source lumineuse D65, est compris dans une plage de 50° à 80°.

4. Produit cosmétique pour maquillage de base selon l'une quelconque des revendications 1 à 3, dans lequel le produit cosmétique pour maquillage de base est une poudre compressée, et un angle de teinte h d'une couleur apparente du produit cosmétique pour maquillage de base, mesuré avec une lumière standard provenant d'une source lumineuse D65, est compris dans une plage de -90 ° à 90 °.

5. Procédé de fabrication d'un produit cosmétique pour maquillage de base, le procédé comprenant les étapes consistant à :
préparer une bouillie en mélangeant un solvant et une matière de base cosmétique comprenant un oxyde rouge de fer et une poudre composite rouge présentant une structure composite, où un pigment rouge sélectionné parmi le groupe consistant en du rubis lithol B et du rubis lithol BCA est intercalé dans une substance inorganique, et
remplir la bouillie dans un récipient, puis éliminer le solvant, dans lequel le solvant est de l'éthanol, de l'eau ou une combinaison de ceux-ci, et dans lequel le spectre de réflexion d'une couleur apparente du produit cosmétique pour maquillage de base présente une valeur minimale locale dans une région de longueurs d'onde allant de 500 à 600 nm

6. Procédé de fabrication d'un produit cosmétique pour maquillage de base selon la revendication 5, dans lequel la poudre composite rouge est une poudre composite rouge, où les valeurs maximales locales respectives d'absorbance mesurées dans une région de longueurs d'onde allant de 400 nm à 600 nm de filtrats obtenus dans le premier essai de dissolution suivant dans l'eau et le second essai de dissolution suivant dans l'éthanol, à l'aide de la poudre composite rouge, sont inférieures à 0,10,
dans lequel, dans le premier essai de dissolution :
1 partie en masse de la poudre composite rouge est dispersée dans 99 parties en masse d'eau distillée sous la forme d'un milieu de dispersion en secouant pendant 60 secondes, la dispersion est ensuite agitée avec un agitateur à 400 tours/minute et 40 °C pendant 6 heures, puis est filtrée par un filtre d'acétate de cellulose présentant un diamètre de pore de 0,45 µm afin d'obtenir un filtrat, et une absorbance du filtrat obtenu est mesurée à l'aide de l'eau distillée comme blanc afin de déterminer une valeur maximale locale d'absorbance dans une région de longueurs d'onde allant de 400 nm à 600 nm, et
dans lequel, dans le second essai de dissolution :
1 partie en masse de la poudre composite rouge est dispersée dans 99 parties en masse de 99,5 v/v % d'éthanol absolu en secouant pendant 60 secondes, la dispersion est ensuite agitée avec un agitateur à 400 tours/minute et 40 °C pendant 6 heures, puis est filtrée par un filtre d'acétate de cellulose présentant un diamètre de pore de 0,45 µm afin d'obtenir un filtrat, et une absorbance du filtrat obtenu est mesurée à l'aide de l'éthanol comme blanc afin de déterminer une valeur maximale locale d'absorbance dans une région de longueurs d'onde allant de 400 nm à 600 nm.

7. Procédé de fabrication d'un produit cosmétique pour maquillage de base selon soit la revendication 5, soit la revendication 6, dans lequel le produit cosmétique pour maquillage de base est un fond de teint pulvérulent, et un angle de teinte h d'une couleur apparente du produit cosmétique pour maquillage de base, mesuré avec une lumière standard provenant d'une source lumineuse D65, est compris dans une plage de 50 ° à 80 °.

8. Procédé de fabrication d'un produit cosmétique pour maquillage de base selon l'une quelconque des revendications 5 à 7, dans lequel le produit cosmétique pour maquillage de base est une poudre compressée, et un angle de teinte h d'une couleur apparente du produit cosmétique pour maquillage de base, mesuré avec une lumière standard provenant d'une source lumineuse D65, est compris dans une plage de -90 ° à 90 °.
